# EUROPEAN PATENT APPLICATION

(11) **EP 1 398 014 A2**
(43) Date of publication of application: **17.03.2004**
(21) Application number: 03003627.1
(22) Date of filing: 18.02.2003
(51) Int. Cl.: A61F 13/42

(54) **Diaper having an electronic wetness indicator system and its manufacturing method**

(30) Priority: 27.08.2002 EP 02019216
(71) Applicant: Huang, Chien-Tung, Hua-Lien County (TW); Chuan, Lin Wen, Hsin Chuang City, Taipei County (TW)
(72) Inventor: Huang, Chien-Tung, Hua-Lien County (TW); Chuan, Lin Wen, Hsin Chuang City, Taipei County (TW)
(74) Representative: Onn, Thorsten

(57) **Abstract**

A manufacturing method of an electronic diaper is to provide a sensor feeding band or box supplying a diaper body with a sensor which will be in advance attached on the upper or the lower dry layer of the diaper body for carrying out a sequent process of automatically manufacturing an electronic diaper. The electronic diaper includes an upper and lower dry layer and an sbsorbing layer sandwiched between the two dry layers, a sensor attached on the upper or the lower dry layer. The sensor has a connecting member for connecting with a controller. Sensing the diaper body wetted, the sensor will give a signal to the controller which will immediately give out warning signals to remind a nurse that the diaper is wet and needs to be replaced with a new one so that a user may feel comfortable in using the electronic diaper.

## Description

### BACKGROUND OF THE INVENTION

This invention relates to an manufacturing method for an electronic diaper and its structure, particularly to one able to give out a warning signal to remind a nurse that the diaper is wet and needs to be replaced with a new one.

Some of the conventional diapers on the market are respectively provided with water-sensitive ink on its outer waterproof layer, which can sense wetness in the waterproof layer of a diaper and change its color for indicating that the diaper has become wet.

However, such a conventional diaper has the following defects.
1. Although the water-sensitive ink may change its color when the waterproof layer of a diaper becomes wet, yet the diaper has only one fine color-changing strip attached on its outer surface so the color change of the color-changing strip can not be seen manifestly. In this case, a nurse usually has to pull down a litle a user's trousers first in order to check clearly if the color change of the diaper happens and decide whether it should be replaced or not. But sometimes a nurse may hardly recognize it even the diaper becomes wet by observing the color-changing strip in a dim room, causing much trouble and inconvenience.
2. Whether a diaper becomes wet or not is simply judged by observing the color change of the color-changing strip. Thus, if a hired nurse pays no attention to it as he/she should do, or even does not replace the wetted diaper, an employer can hardly find any specific evidence to prove that the nurse neglects his/ her work on purpose.
3. The color-changing strip of a conventional diaper is liable to be affected by the air humidity, that is, when there is too much air humidity, the water-sensitive ink of the color-changing strip will sense the humidity and change its color, easily resulting in wrong judgment of a nurse.

Other conventional electronic diapers, disclosed in Taiwan patents No.312134, 388712 and 422088, are respectively embedded inside with an electric conduction net or a metallic foil to sense wetness in a diaper. To combine an electric conduction net or a metallic foil with a diaper is practicable in theory but difficult in an automatic manufacturing process, so the foresaid conventional electronic diaper can hardly be produced automatically.

Besides, the conventional electronic diapers, disclosed in Taiwan patents No. 312134 and 422088, respectively employ a comparison circuit as a control circuit, not only increasing producing coat, but also consuming too much electricity and necessary to replace its battery after used for only a short period of time.

The conventional electronic diaper, disclosed in Taiwan patent No. 388172, transmits signal by means of an emission and reception circuit, increasing producing cost, economically not profitable and consuming too much electricity.

### SUMMARY OF THE INVENTION

One objective of the invention is to offer an electronic diaper capable of showing the time of wetting of a diaper and giving out a sensing out wetness of a diaper body and producing signal to remind a nurse that the diaper is wet and has to be replaced with a new one, heightening a nurse's sense of duty and letting a user feel comfortable in using the diaper.

A second objective of the invention is to offer an electronic diaper having a sensor supplied by a sensor feeding strap or box and attached in advance on the inner surface of the upper or the lower layer of a diaper for facilitating automatically producing electronic diapers.

A third objective of the invention is to offer an electronic diaper having a diaper body, a sensor and a controller connected with the sensor for mutual operation, and the sensor placed in the diaper for sensing wetness of the diaper body so that the sensor may be discarded after used or separated from the diaper body to be repeatedly used, lowering cost in use.

The features of the invention are as follows.
1. The electronic diaper of the invention is provided inside with a sensor, which is possible to extend to the front or the rear side of the diaper and has one end provided with a connector for connecting the sensor with a controller.
2. The manufacturing method of an electronic diaper in the present invention is to have an automatic sensor feeding band supplying a diaper with a sensor, which is to be attached on the inner surface of the upper or the lower dry layer of the diaper, able to produce electronic diapers automatically with a lower cost.
3. The electronic diaper of the invention is provided with a controller able to be used repeatedly, lowering cost in using and manufacturing the electronic diaper.
4. The sensor of the electronic diaper of the invention is able to operate mutually with a controller, and the sensor placed in a diaper body for sensing wetness of the diaper body can be discarded after used once or many times.
5. The sensor of the invention is in advance printed with conductive lines on the upper surface of its lower layer film, and/or has an upper layer film pasted on the upper surface of the lower layer film to making up the sensor.
6. The controller in the invention can supply the sensor with continual or intermittent power so as to check a signal coming from the sensor of the wet condition of the diaper body, and cuts off the power to the sensor immediately once it finds out that the diaper body is already wet enough to be replaced with a new one, getting rid of anxiety of a user of the electronic diaper.

### BRIEF DESCRIPTION OF DRAWINGS

This invention will be better understood by referring to the accompanying drawings, wherein:
Fig. 1 is a cross-sectional view of a first preferred embodiment of an electronic diaper in the present invention:
Fig. 2 is a partial perspective and cross-sectional view of a second preferred embodiment of an electronic diaper in the present invention:
Fig. 3 is a partial perspective and cross-sectional view of a third preferred embodiment of an electronic diaper in the present invention:
Fig. 4 is a partial perspective and cross-sectional view of a fourth preferred embodiment of an electronic diaper in the present invention:
Fig. 5 is a perspective view and a partial magnified view of the first preferred embodiment of the sensor of an electronic diaper in the present invention:
Fig.6 is a perspective view of the first preferred embodiment of a sensor, and a perspective and a partial magnified view of the first preferred embodiment of a controller of the electronic diaper in the present invention:
Fig. 7 is a perspective and a partial magnified view of a second preferred embodiment of the sensor of the electronic diaper in the present invention:
Fig.8 is a perspective view of a second preferred embodiment of the sensor, and a perspective and a partial magnified view of a second preferred embodiment of the controller of the electronic diaper in the present invention:
Fig. 9 is a perspective and a partial magnified view of a third preferred embodiment of the sensor of the electronic diaper in the present invention:
Fig. 10 is a perspective view of the third preferred embodiment of the sensor, and a perspective and a partial magnified view of a third preferred embodiment of the controller of the electronic diaper in the present invention:
Fig. 11 is a perspective view of a fourth preferred. embodiment of the sensor, and a perspective and a partial magnified view of a fourth preferred embodiment of the controller of the electronic diaper in the present invention:
Fig. 12 is a perspective view of a fifth preferred embodiment of the sensor, and a perspective and a partial magnified view of a fifth preferred embodiment of the controller of the electronic diaper in the present invention.
Fig. 13 is a perspective view of a first preferred embodiment of a manufacturing method of the electronic diaper in the present invention:
Fig. 14 is a perspective view of a second preferred embodiment of a manufacturing method of the electronic diaper in the present invention:
Fig. 15 is a perspective view of a third preferred embodiment of a manufacturing method of the electronic diaper in the present invention:
Fig. 16 is a perspective view of a fourth preferred embodiment of a manufacturing method of the electronic diaper in the present invention:
Fig. 17 is a perspective view of a preferred embodiment of the controller, the sensor and the diaper body separated from one another in the present invention:
Fig. 18 is a perspective view of a preferred embodiment of a wired display connected with the electronic diaper in the present invention:
Fig. 19 is a perspective view of a preferred embodiment of a wireless display of the electronic diaper in the present invention:
Fig. 20 is a diagram of a first preferred embodiment of a group display of the electronic diaper in the present invention:
Fig. 21 is a diagram of a second preferred embodiment of the group display of the electronic diapers in the present invention:
Fig. 22 is a flowing chart of the operation of the software of an IC in the controller of the electronic diaper in the present invention:
Fig. 23 is a diagram of a first preferred embodiment of the control circuit of the controller of the electronic diaper in the present invention, and,
Fig. 24 is a diagram of a second preferred embodiment of the control circuit of the controller in present invention.

### DETAILED DESCRIPTION OF THE PREFERRED

### EMBODIMENT

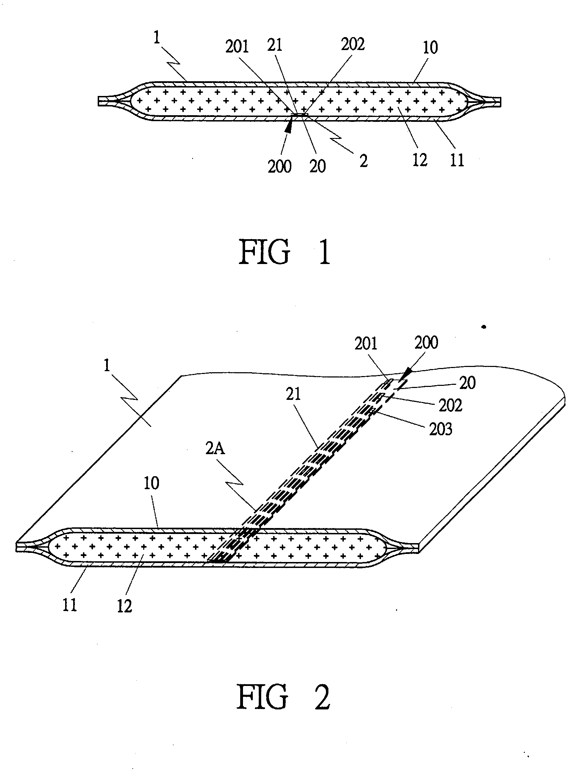

A first, a second, a third and a fourth preferred embodiment of an electronic diaper in the present invention, as shown in Figs. 1-4, respectively includes a diaper body 1 and a sensor 2.

The diaper body 1 is provided with an upper and a lower dry layer 10, 11, and an absorbing layer 12 sandwiched between the two dry layers 10, 11. The sensor 2 (2A; 2B ; 2C) is disposed on the upper dry layer 10 and/or on the lower dry layer 11.

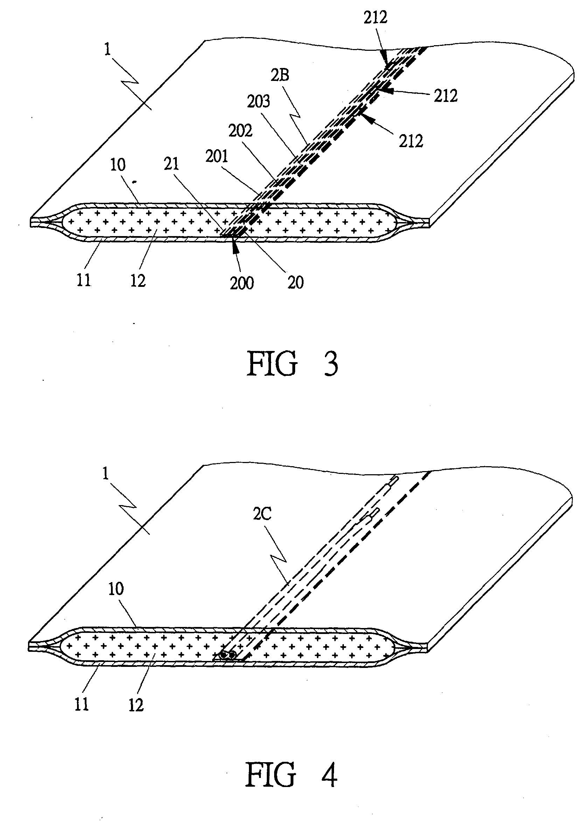

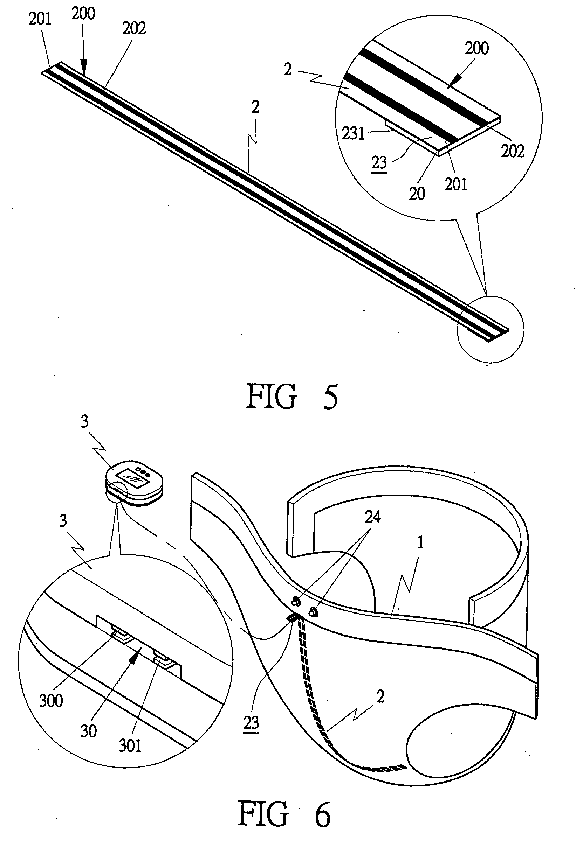

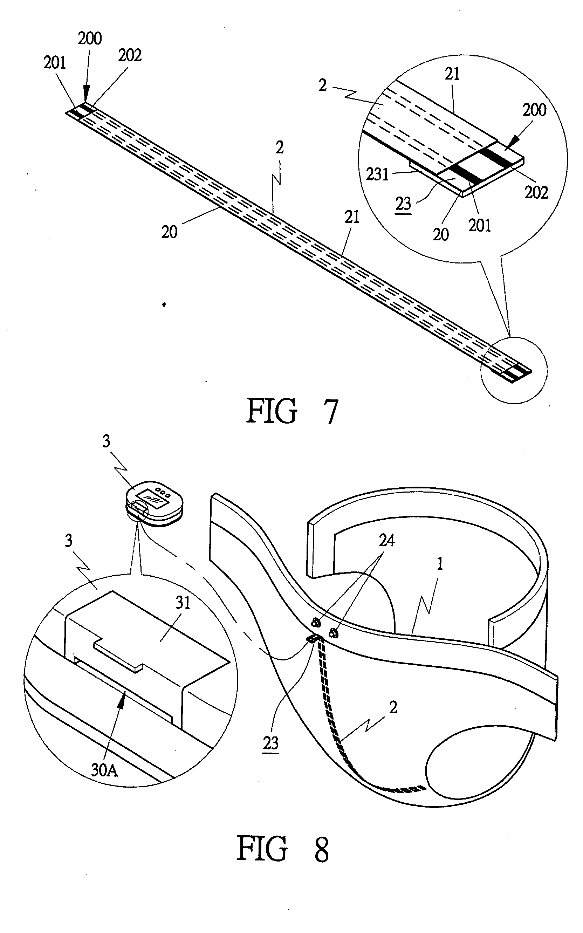

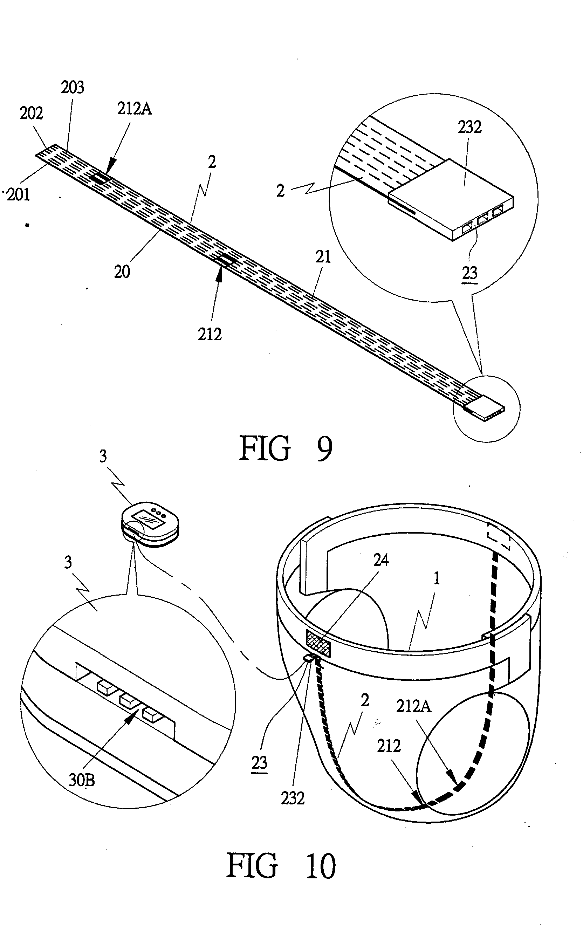

The sensor 2 (2A; 2B; 2C), as shown in Figs. 1-3, is formed with a lower film layer 20 made of plastic, rubber or silicon rubber. The lower film layer 20 is in advance provided on its upper surface 200 with two electric conductors 201, 202 to function as the sensor 2, as shown in Fig. 1, or three electric conductors 201, 202, 203, as shown in Figs. 2 and 3, or a plurality of electric conductors. An upper film layer 21 made of plastic, rubber or silicon rubber respectively matching with the material of the lower film layer 20 is pasted closely on the upper surface 200 of the lower film layer 20 of the sensor 2 (2A, 2B) to make up a sensor 2 soft and having good conductivity, as shown in Figs. 7 and 9, but the upper film layer 21 is not necessarily needed, as shown in Fig. 5. And the sensor 2C is formed with a conductive line to attain its object.

Thus, the sensors 2 (2A, 2B, 2C) respectively have the lower or the upper surface coated with glue to be combined with the diaper body 1 by means of automatic equipment.

The two electric conductors 201, 202, or the three electric conductors 201, 202, 203 of the sensor 2 (2A, 2B, 2C) can become conductive to each other by wetness in the diaper body 1 to generate trigger signals. The sensors 2 (2A, 2B, 2C) can be disposed on the outer side of the diaper body 1, either at a front side, as shown in Figs. 6, 8 and 11, or a rear side or both a front and a rear side, as shown in Fig. 10, or a side portion, depending on a user's posture of lying in bed. Besides, the sensor 2 (2A, 2B, 2C) has the two or the three or more electric conductors respectively formed with exposed ends to carry out sensing of wetness in the diaper 1, as shown in Fig. 2, or has the upper film layer 21 or the lower film layer 20 bored with holes 212 to let urine in the diaper body 1 to flow through to contact the electric conductors to electrically connected to each other for generating trigger signals of wetness of the diaper body 1.

In order to connect the sensor 2 with a controller 3, the sensor 2 (2A, 2B, 2C) is in advance provided at one end with a connecting member 23 able to be inserted in a socket hole 30 of the controller 3. Providing modes of the connecting 23 with the sensor 2 (2A, 2B, 2C) are shown in Figs. 5-11.

Figs. 5 and 6 show that one end of the sensor 2 (2A; 2B; 2C) has a hard film 231 to function as the connecting member 23 able to be directly inserted in the socket hole 30 of the controller 3, letting the two electric conductors 201, 202 of the sensor 2 (2A, 2B) respectively clamped by and contact with terminals 300, 301 fixed in the insert hole 30 of the controller 3, and then the sensing film 2 (2A; 2B; 2C) is connected with the controller 3.

Figs. 7 and 8 show that the sensor 2 (2A; 2B; 2C) has one end provided thereon with a hard film 231 to function as a connector 23 able to be inserted in the socket hole 30A of the controller 3, letting the terminals in the insert hole 30A contact with the two electric conductors 201, 202 of the sensor 2, and then the controller 3 is connected with the sensor 2 (2A; 2B; 2C).

Figs. 9 and 10 show that the sensor 2 (2A; 2B; 2C) has one end provided with a quick plug 232 to function as a connecting member 23 able to be inserted in the socket hole 30B of the controller 3, able to combine the controller 3 with the sensor 2 (2A; 2B; 2C) with quickness.

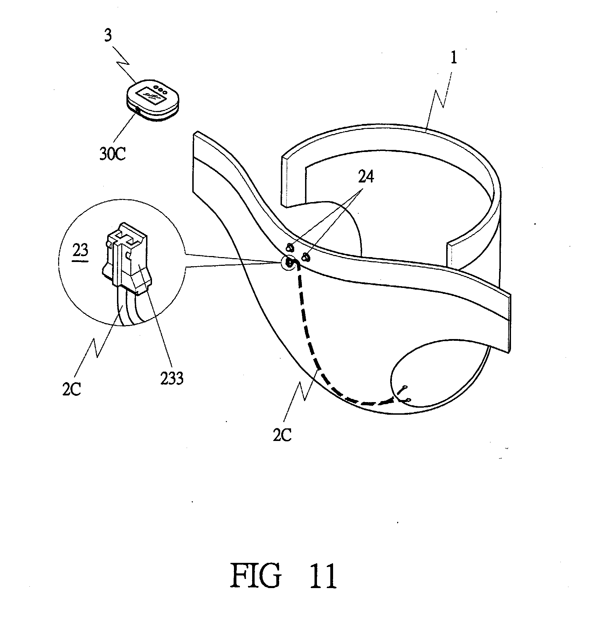

Fig. 11 shows that the sensor 2C has one end provided with a quick plug 233 to function as a connecting member 23 able to be inserted in the socket hole 30C of the controller 3, able to combine the controller 3 with the sensor 2C quickly.

Thus, when a diaper body 1 has become wet with urine, the controller 3 can quickly be detached from the wetted diaper body 1 and then combined with the sensor 2 (2A, 2B, 2C) of a new diaper body 1, enabling the controller 3 to be used repeatedly.

Further, a fixing member 24, like a fastener, as shown in Figs. 6, 8 and 11, or a Velcro band, as shown in Fig. 10, is provided on the front or the rear side of the diaper 1 for attaching or pulling off the controller 3 conveniently.

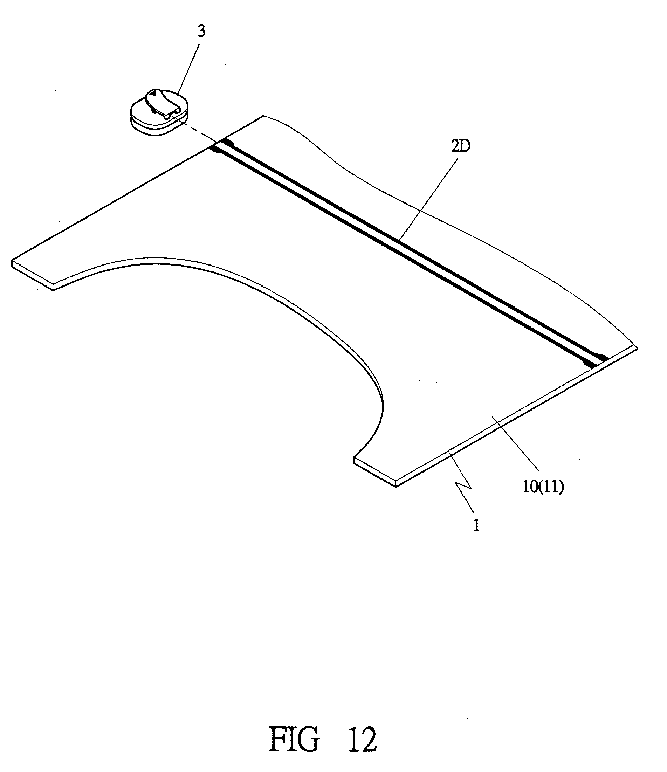

Next, Fig. 12 shows a fifth embodiment of an electronic diaper in the present invention, which includes a diaper body 1 consisting of an upper dry layer 10, a lower dry layer 11, a sucking layer 12 sandwiched between the two dry layers 10 and 11, and a sensor 2D printed on the upper dry layer 10 and/or the lower dry layer 11. The sensor 2D has the same function as those 2A, 2B, 2C in the four embodiments described above, and the front end of the diaper body 1 can be combined with the controller 3, which is then connected with the sensor 2D and receives signals from the sensor 2D to accordingly give out warning signals.

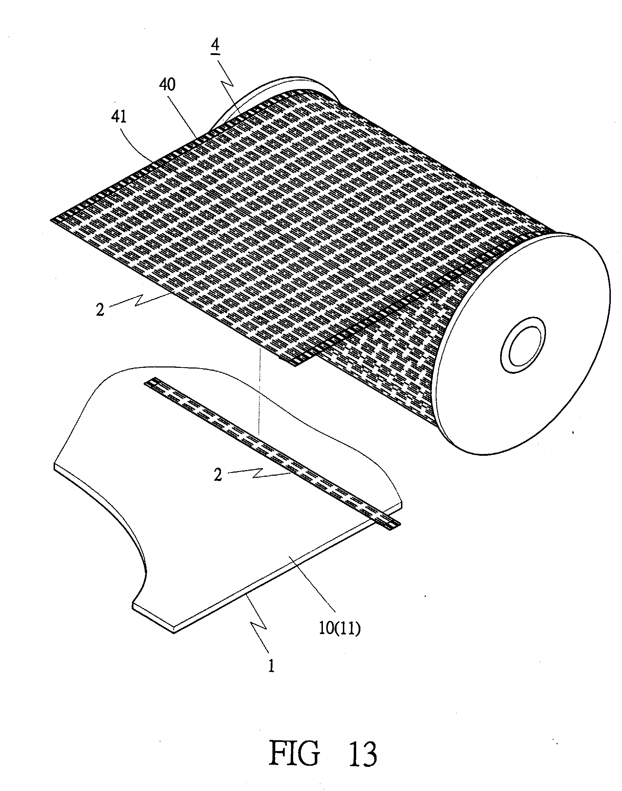

In order to manufacture electronic diapers automatically, a sensor-feeding band 4 is provided for supplying the diaper body 1 with the sensor 2, as shown in Figs. 12 and 13. The sensor feeding band 4 is a continuous band body 40 having a large number of sensors 2 (2A, 2B, 2C) spaced apart equidistantly thereon, able to be peeled off and adhered on the upper or the lower dry layer 10, 11 of a diaper 1 by means of automatic equipment. Besides, a tear line 4 is formed between every two abutting sensors 2 (2A, 2B, 2C). Thus, the sensor 2 (2A, 2B, 2C) can be stuck on the inner surface of the upper or the lower dry layer 10, 11 of the diaper body 1 with glue or double-side adhesive tape, or by fusing and adhering carried out by a high frequency machine, thus facilitating automatically manufacturing the diaper 1.

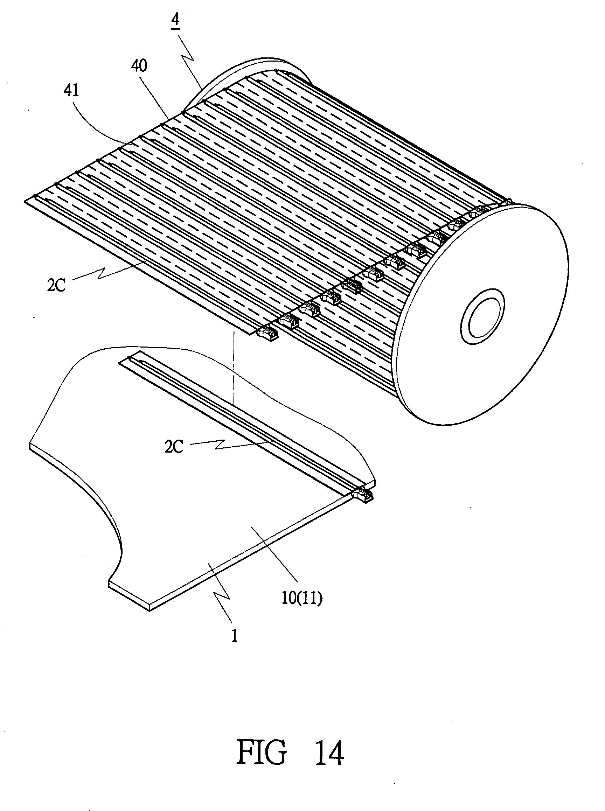

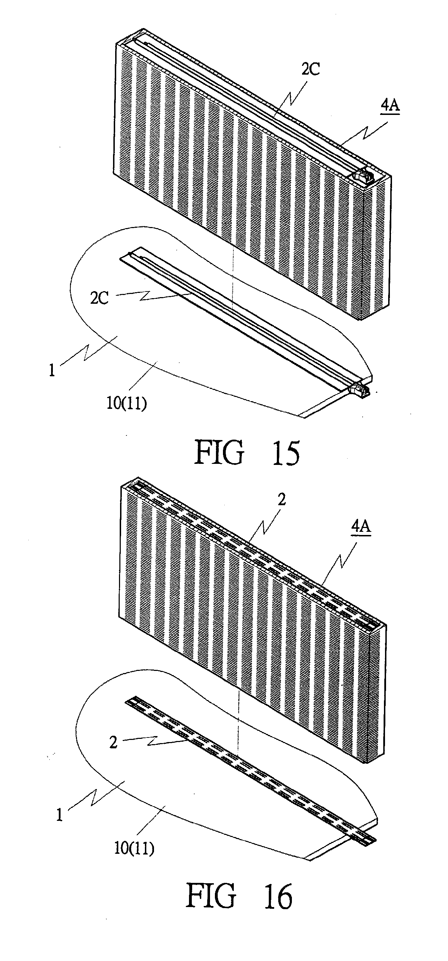

Moreover, a sensor feeding box 4A, as shown in Figs. 14 and 15, is provided therein with numerous sensors 2 (2A; 2B; 2C) piled on one another. The sensor 2 (2A; 2B; 2C) is to be attached on the inner surface of the upper or the lower dry layer 10, 11 of the diaper body 1 with glue or double-side adhesive tape, or by fusing and adhering carried out by a high frequency machine with the help of automatic equipment, thus convenient for automatically manufacturing the diaper body 1.

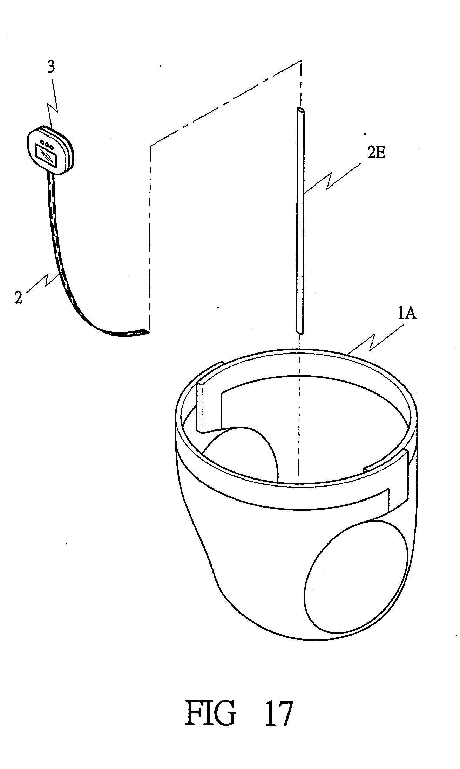

As described above, the sensor 2 (2A, 2B) is combined with the diaper body 1 in advance and then can be thrown away together with the used diaper body 1 after used. In addition, the controller 3, the sensor 2 (2A, 2B) and the diaper 1A can be detached, convenient for a user, a nurse or an attendant in use, saving its cost, and avoiding wetness of the diaper body and bedsore. A shown in Fig. 17, the sensor 2 (2A; 2B; 2C) is placed in the diaper body 1A before or after the diaper body 1A is wrapped around the body of a user, and the sensor 2 (2A; 2B; 2C) may have its outer side wrapped with an absorbing material 2D, such as technical fabric or absorbing cotton or paper. Thus, the sensor 2 (2A; 2B; 2C ) can be used repeatedly after cleaned and sterilized, economically saving.

Various applications of the electronic diaper in the present invention are respectively shown in Figs. 17, 18, 19 and 20. Fig. 17, showing that the controller 3 is connected to a wired outer hanging display 33, which can be placed where a nurse can check it easily, such as being hung on a user's chest or other places convenient for checking the outer hanging display 33.

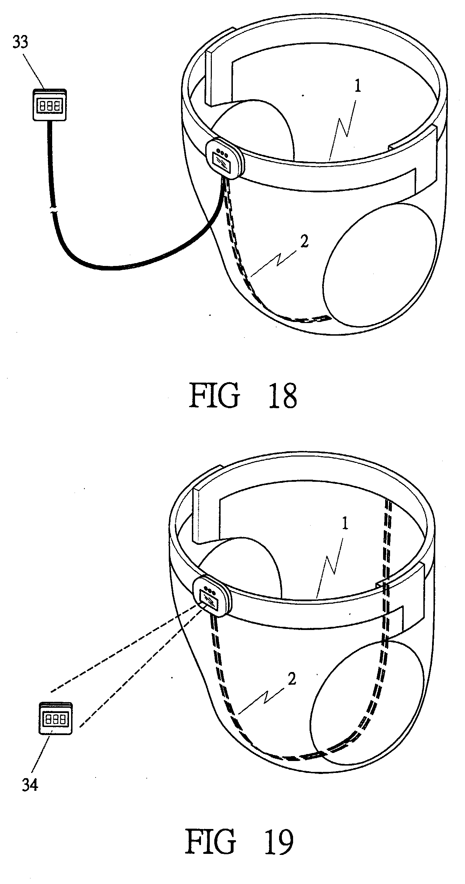

Fig. 19 shows that the controller 3 is provided inside with an emitter to cooperate with a wireless outer hang display 34 having a receptor, which is carried with a nurse, who may carry out her responsibility without fail.

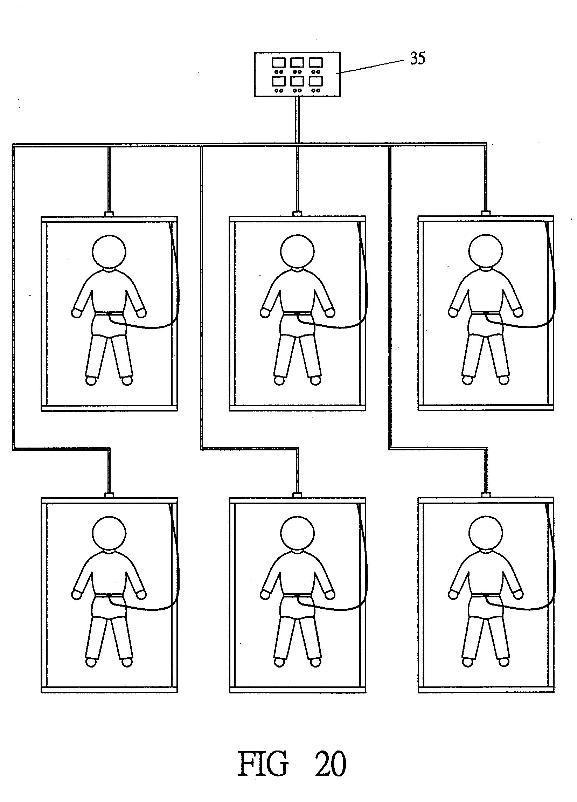

Fig. 20 shows that the controller 3 is connected to a group outer hanging display 35, and this is applicable to hospitals or nursing homes where wireless emission is prohibited. The outer hanging display 35 is placed on a nursing counter to let the nurse know the condition of the diaper body of each user.

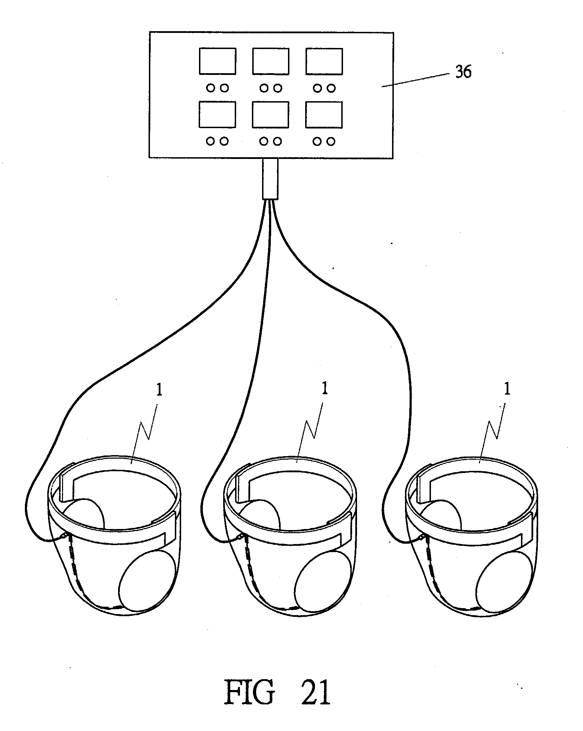

Fig. 21 shows that a plurality of the diaper bodies 1 are directly connected to a group outer hanging display 36, and this is applicable to hospitals or nursing homes where wireless emission is forbidden. The outer hanging display 36 is placed on a nursing counter to let the nurse know the condition of every diaper body 1.

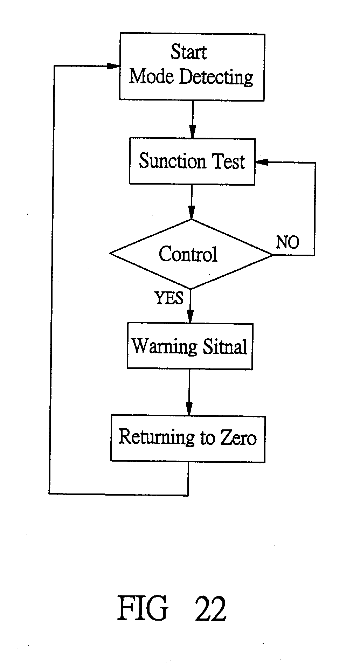

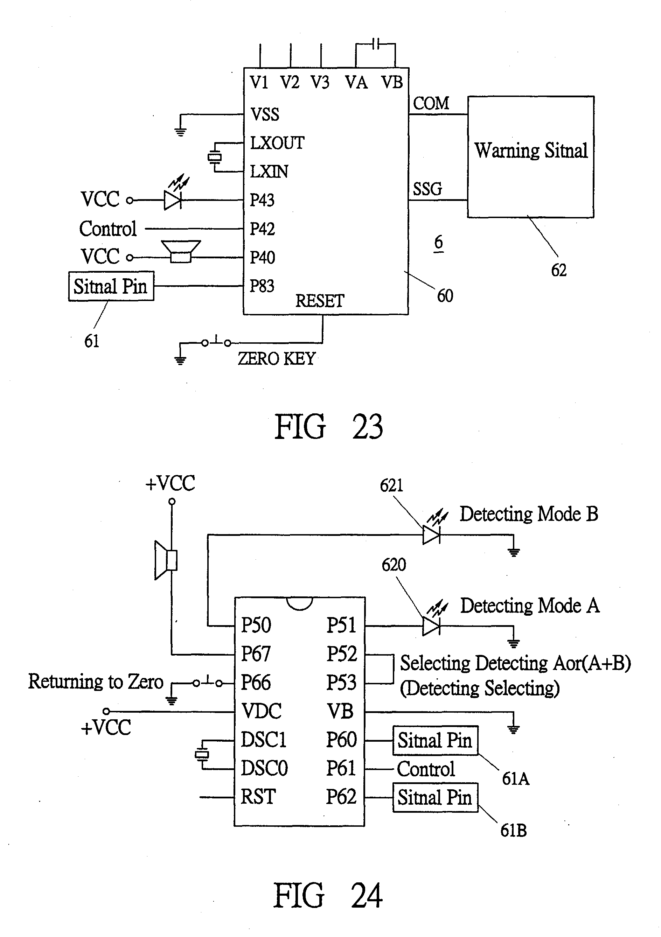

In addition, the controller 3 can be provided inside with a control circuit 6, as shown in Fig. 23 or 24. The control circuit 6 is constructed of an integrated circuit (IC) 60, as shown in Fig. 22, and the IC 60 having a drive program burned inside is connected with the sensor 2 (2A, 2B, 2C) for transmitting a signal 61 sensed by the sensor 2 (2A; 2B; 2C), which is calculated and converted by the IC 60 into warning signals 62. The warning signal can be turned into a light given out by a LED lamp 63 or a sound given out by a horn, and/or the time of wetting of the diaper body 1 by a liquid crystal display provided. Thus, when the diaper body 1 becomes wet but is not yet replaced with a new one, the horn will in due time give out a sound to warn a nurse or an attendant to replace the diaper body 1, preventing the nurse from not replacing the diaper body 1 on purpose, elevating a nurse's sense of duty and letting the user feel comfortable in using the electronic diaper.

Additionally, the controller 3 may enable the sensor 2 as a signal source 61 carry out sensing continuously, that is, the controller 3 supplies continuously or intermittently the sensor 2 (2A, 2B, 2C) with a very small and incessant power for sensing the condition of the diaper body 1. That is, the controller 3 supplies the sensor 2 (2A, 2B, 2C) with a very small power at short intervals of time (seconds or minutes) so long as it can sense the condition of the diaper body 1 at a proper time.

The control circuit 6 of the controller 3, as shown in Fig. 24, is provided with two signal sources 61A, 61B and has two sensing modes A, B. The two signal sources 61A, 61B conform to the structure of the diaper body 1 shown in Fig. 2, which is provided with the sensor 2 (2A; 2B; 2C; 2D) having three electric conductors 201, 202, 203. The electric conductors 201 and 202 are comparatively short and of a same length, while the electric conductor 203 is comparatively longer then the conductors 201, 202, with the electric conductor 202 used as a mutual grounding line. Thus, the two conductors 201, 201 can be electrically connected by urine flowing through a hole 212 bored in the upper film layer, so the sensor 2 can sense out a first stage of wetting condition of the diaper body 1 to produce a first wetting signal 61A when there is only a little wetness in the diaper body 1. The two conductors 202, 203 may be electrically connected by urine flowing through a hole 212A of the upper film layer, sensing a second stage of wetting condition of the diaper body 1 and producing a second wetting signal 61B of the diaper body, when there is enough urine in the diaper body 1. Further, the first wetting signal 61A may enable the controller 3 to turn on a green light of the LED 620, indicating that the diaper body 1 is not yet needed to be replaced with a new one. The second wetting signal 61B may enable the controller 3 turn on the red light of the LED 621, indicating that the diaper body 1 has to be replaced with a new one. Or the through conductors 201, 202, and 203 are made to have different lengths, with the conductor 202 functioning as a grounding line, then they can form plural signal sources for replacing the diaper body 1 in due time.

The operation of the integrated circuit 60 in the present invention, as shown in Fig. 22, is that when power is started, the integrated circuit 60 will start mode detecting to check the system condition and carry out function testing. Then, the controller 3 supplies the sensor 2 (2A; 2B; 2C; 2D) of the diaper body 1 with a continuous or an intermittent power for sensing the signal source 61 of the condition of the diaper body 1. In case the signal source 61 shows yes, it indicates that the diaper body 1 has become wet enough, and at this time the controller 3 will immediately cut off the power of the sensor 2 (2A; 2B; 2C; 2D), but the warning signal of the controller 3 will be continuously given out until the diaper 1 is replaced with a new one, and the signal source 61 gets into a zero position to force the controller 3 into a stand-by condition to wait for a next signal source 61, thus ensuring safety in using the electronic diaper of this invention.

While the preferred embodiments of the invention have been described above, it will be recognized and understood that various modifications may be made therein and the appended claims are intended to cover all such modifications that may fall within the spirit and scope of the invention.

## Claims

1. A manufacturing method of an electronic diaper comprising provision of a sensor feeding band, said sensor feeding band having a continuous band body, said continuous band body having a plurality of sensor bodies spaced apart equidistantly with a tear line formed between every two abutting ones of said sensors, each said sensor able to be torn off along said tear line, said sensor attached on an inner surface of an upper or a lower dry layer of a diaper body with glue or by fusing and adhering operated by a high frequency machine so as to facilitate automatically manufacturing said electronic diaper.

2. The manufacturing method of electronic diaper as claimed in Claim 1, wherein a sensor feeding box is provided for placing numerous sensors piled on one another in its interior, and each said sensor is attached by automatic equipment on the inner surface of the upper or the lower dry layer of each said diaper body with glue or by fusing and adhering operated by a high frequency machine to conveniently and automatically manufacturing said electronic diaper.

3. An electronic diaper comprising a diaper body, said diaper body provided at least with an upper and a lower dry layer, an absorbing layer sandwiched between said two dry layers, said upper or lower dry layer attached with a sensor, said sensor able to extend to an outer side of said diaper, said sensor having a connecting member.

4. The electronic diaper as claimed in Claim 3, wherein said sensor is formed with a lower film layer having plural electric conductors disposed on its upper surface, with an upper film layer coated closely on the upper surface of said lower film layer to make up said sensor soft and having good conductivity.

5. The electronic diaper as claimed in Claim 3, wherein said sensor is formed with a lower film layer provided on its upper surface with plural electric conductors, becoming soft and having good conductivity.

6. The electronic diaper as claimed in Claim 3, wherein an outer hang display is provided to be connected with said diaper body and placed at a place where an attendant can check it easily.

7. An electronic diaper comprising:
a diaper body provided with an upper day layer, a lower day layer and an absorbing layer sandwiched between said upper and said lower dry layer:
a sensor attached on said upper dry layer or on said lower dry layer and having a connecting member; and,
a controller having a socket hole with two terminals provided in its interior, said connecting member of said sensor inserted in said socket hole of said controller so that said controller may receive signals from said sensor for producing warning signals of the condition of said diaper body.

8. The electronic diaper as claimed in Claim 7, wherein said controller can clasp conductors of said sensor for receiving signals from said sensor and accordingly produces warning signals of the witted condition of said diaper body.

9. An electronic diaper comprising:
a diaper consisting of an upper dry layer, a lower dry layer and an absorbing layer sandwiched between said two dry layers;
a controller having a socket hole provided with two terminals in its interior;
a sensor having a connecting member, said connecting member inserting in said socket hole of said controller, said sensor able to be attached in said diaper body for sensing the condition of said diaper body.

10. The electronic diaper as claimed in Claim 9, wherein said sensor is wrapped with an absorbing material.

11. An electronic diaper comprising
a diaper provided at least with an upper dry layer, a lower dry layer and an absorbing layer sandwiched between said two dry layers,
a plurality of conductors of said sensor printed on said upper dry layer and/or said lower dry layer, said sensor able to be connected with a controller so that said controller may receive signals from said sensor to produce warning signals of the condition of said diaper body.

12. An electronic diaper comprising;
a diaper body consisting of an upper dry layer, a lower dry layer, and an absorbing layer sandwiched between said two dry layers;
a sensor attached on said upper dry layer or said lower dry layer, said sensor connected with a controller;
said controller provided with a control circuit in its interior, said control circuit constituted with an IC, said IC burned with a driving program, said controller receiving signals sensed by said sensor, said controller
Calculating and converting said signals into warning signals, which is then transmitted to an LED lamp to be turned on to light up, or to a buzzer to give out sound, or to an LCD display to indicate the time of said diaper body becoming wet so that said diaper body may be replaced with a new one by an attendant.

13. The electronic diaper as claimed in Claim 12 wherein said control circuit has a plurality of signal sources and plural detecting modes in correspondence with said sensor consisting of three conductors; two of said three conductors are electrically connected with each other by urine flowing through a hole formed in an upper film layer to sense a first stage of wetting condition of said diaper body and producing a first signal of wetting condition of said diaper body to be transmitted to said controller, which then produces a first warning signal; the other two of said three conductors may be electrically connected by urine flowing through another hole formed in said upper film layer to sense a second stage wetting condition of said diaper body and to produce a second signal of wetting condition of said diaper body so that said controller may receive said second signal to produce a second warning signal for attaining the object of replacing said diaper body with a new one.

14. The electronic diaper as claimed in Claim 12, wherein said controller supplies said sensor with a very small and continuous power for said sensor to sense the condition of said diaper continuously; or said controller supplies said sensor with a very small and intermittent power for said sensor to carry out sensing the condition of said diaper intermittently, and once wetness of said diaper is sensed, said controller will immediately cut off the power of said sensor, but said warning signal is still given out until said diaper is replaced with a new one.
